Europäisches Patentamt

(19) European Patent Office

Office européen des brevets

(11) Publication number: **0 368 624**
**A2**

(12) # EUROPEAN PATENT APPLICATION

(21) Application number: **89311520.4**

(22) Date of filing: **07.11.89**

(51) Int. Cl.5: **G01N 33/543, G01N 33/546,**
**//G01N33/94,G01N33/576**

(30) Priority: **09.11.88 US 269623**

(43) Date of publication of application:
**16.05.90 Bulletin 90/20**

(84) Designated Contracting States:
**AT BE CH DE ES FR GB GR IT LI LU NL SE**

(71) Applicant: **Biotrack, Inc.**
**1058 Huff Avenue**
**Mountain View California 94043(US)**

(72) Inventor: **Gibbons, Ian**
**1003 Fremont Street**
**Menlo Park California 94025(US)**
Inventor: **Hsu, Po Choo**
**21020 Classic Court**
**Cupertino California 94014(US)**
Inventor: **Smoluk, Geraldine D.**
**1090 Pomeroy Avenue**
**Santa Clara California 95051(US)**

(74) Representative: **Harrison, David Christopher**
**et al**
**MEWBURN ELLIS 2 Cursitor Street**
**London EC4A 1BQ(GB)**

(54) **Method and composition for stabilizing and solubilizing latex reagents.**

(57) A method for providing dry, resuspendible, stable latex particles on a surface is disclosed, which comprises applying the particles to the surface in the presence of a water soluble, solid, polyhydroxy sugar or sugar alcohol. The method is particularly applicable to the use of devices with internal chambers having small volumes and in which the liquid diluent for the dry reagent enters a chamber containing the latex particles without mixing or passes over a track containing the latex particles.

EP 0 368 624 A2

## METHOD AND COMPOSITION FOR STABILIZING AND SOLUBILIZING LATEX REAGENTS

This invention is related to methods of stabilizing and solubilizing latex reagents, particularly latex reagents having antibodies or antigens on their surfaces and which have been dried onto plastic containers or test devices.

A number of small diagnostic devices have been developed in the laboratories of the inventors over the past several years. Typical of these are the devices described in US Patent Application Serial Nos. 07-016,506 (February 17, 1987), 06-880,793 (July 1, 1986; now USP 4,756,884), 07-090,026 (August 27, 1987), and 07-117,791 (November 5, 1987). All of these applications describe devices made of plastic that contain capillary chambers and channels (as well as non-capillary spaces in some cases) used to automatically sample biological fluids, such as blood or urine, and mix them with reagents for detection of an analyte. These devices have provided patient-side analysis of a number of analytes in a convenient manner.

However, while these devices work well under the circumstances described in the applications, there are a number of difficulties in utilizing the extremely small volumes and plastic surfaces of these devices under certain circumstances. For example, some assay reagents, especially proteins and other biological materials, often tend to stick to plastic surfaces. When the sample is used to resuspend such reagents, the small volumes of sample that are available, particularly in capillary spaces, can lead to problems in the resuspension process.

A particular example is the problem that has led the present invention. It has proven desirable to carry out agglutination assays using latex particles in test devices of the type set forth in the applications identified above. Latex particles are readily available from commercial sources, and techniques for their use in agglutination assays have been well developed. However, latex particles, particularly particles having antibodies or other proteinaceous reagents bound to their surfaces for use in the agglutination process, have proved particularly difficult to stabilize for long-term storage or reconstitute with small sample volumes once they have dried onto plastic surfaces. In particular, non-specific aggregation of particles commonly occurs during the storage or reconstitution process. Since aggregation of particles is similar to the agglutination reaction that would normally indicate the analyte concentration, it is difficult or impossible to routinely distinguish samples that contain analyte from samples that are devoid of analyte.

Accordingly, there remains a need for new methods and compositions that will allow long-term storage of latex particles in plastic devices as well as the rapid and unaggregated resuspension of latex reagents using small volumes of sample after such reagents have been dried onto plastic surfaces.

The present invention provides methods and compositions for use in providing stable storage of latex particles. The invention also provides rapid resuspension of latex particles from plastic surfaces onto which they have been dried. The present method provides a readily resuspendible latex particle composition dried onto plastic surfaces, as well as on other surfaces, in which the composition is prepared by applying the particles to the surface in the presence of a water soluble, solid, polyhydroxy sugar or sugar alcohol. Particularly preferred are $C_4$-$C_7$ monomeric aldose or ketose sugars, dimers or trimers of such sugars, and $C_4$-$C_7$ monomeric fully oxygenated sugar alcohols. The method is particularly applicable to use in an analytical test device comprising a plastic hydrophobic housing and internal chambers capable of containing a reagent or biological sample. The dry latex reagent is present in an internal chamber of such a device, where its readily resuspendible nature allows it to be resuspended without significant inappropriate (non-specific) aggregation when a biological sample enters or passes through the chamber in which the reagent is present. Resuspension readily takes place with a single pass of diluent through a thin chamber of capillary dimensions or by entry of diluent into a chamber with gentle mixing.

## DESCRIPTION OF SPECIFIC EMBODIMENTS

This invention provides improved devices and methods that rely on the aggregation of latex particles for indication of the presence (or absence) of an analyte and for precise determination of the concentration of an analyte in a sample. The invention is particularly directed to devices that rely upon capillaries and other small chambers and orifices to pump fluids; to control measurement of fluids, reaction times, and mixing of reagents; and to determine a detectable signal. Such devices are described in US Patent Application Serial No. 06-880,793, now US Patent No. 4,756,884, and the related applications that have been previously indicated. By providing a water soluble, solid, polyhydroxy sugar or sugar alcohol as a resuspending agent, it is possible to provide latex reagents as stabilized, dry reagents in the small internal chambers and

2

channels of such devices while achieving satisfactory resuspension of the latex particles in a single pass of liquid sample or reagent through a chamber containing the dried latex reagent. Without use of the suspending agents of the invention, unsatisfactory aggregation takes place so that it is not possible to determine an accurate assay result. Additionally, in some cases insufficient resuspension of the reagent takes place in the absence of suspending agents of the invention. The particular requirements of these specialized devices will be discussed further in later sections of this specification, and the characteristics of such devices can be seen in detail in the indicated prior applications and patents.

The present invention is not restricted to methods carried out in or to devices of the type described above, however. Latex reagents can be stabilized in dry form and made more readily resuspendible by including a water soluble, solid, polyhydroxy sugar or sugar alcohol under any circumstances in which stabilized latex reagents are used. The use of sugar and sugar alcohol molecules has proven to be particularly useful in devices of the specific type indicated, as other suspending methods (e.g. sonication) which have proven useful in resuspending dry latex in other types of applications and which eliminate the need for additives cannot be applied successfully to these devices where only relatively weak mixing can be achieved.

The suspending agent of the invention is a water soluble, solid, polyhydroxy sugar or sugar alcohol. Water solubility to provide a solution of at least 5 wt.% preferably 20 wt.%, and more preferably 50 wt.% at 230C is preferred. Solids at room temperature are necessary in order to provide a dry latex reagent. The melting point of the sugar or sugar alcohol should be at least 500C, preferably at least 700C, and more preferably at least 1000C. The compounds are polyhydroxy in that at least two hydroxy groups are present in the sugar or sugar alcohol, preferably at least four, and more preferably four to six hydroxy groups. As is well understood, sugars are compounds containing multiple hydroxy groups and a keto or aldehyde group. Sugar alcohols are molecules that correspond in structure to sugars but which have the keto or aldehyde group reduced to a hydroxy group. Preferred suspending agents are $C_4$-$C_7$ monomeric aldoses and ketoses; dimeric and trimeric sugars comprising $C_4$-$C_7$ monomeric aldoses, ketoses, or mixtures thereof; and $C_4$-$C_7$ monomeric fully oxygenated sugar alcohols. Fully oxygenated sugar alcohols are compounds of the formula $CH_2OH(CHOH)_nCH_2OH$, where n is an integer. In the preferred sugar alcohols indicated above, n is an integer from 2 to 5. Examples of suitable sugars and sugar alcohols include D-turanose, D-maltose, trehalose, sucrose, lactose, D-fructose, mannose, fucose, L-sorbose, D-ribose, 2-deoxy-D-glucose, D-arabinose, D-xylose, glucose, maltotriose, mannitol, sorbitol, ribitol, D-xylitol, L-arabitol, L-xylitol, and D-arabitol. Other compounds that are readily hydrolyzable or otherwise convertible into a sugar or sugar alcohol of the invention when contacted with a biological test fluid are considered to be equivalent to such compounds, and the use of such derivatives that give rise to compounds of the invention in situ are considered to be within the practice of the present invention.

The suspending agent is present in the latex composition in an amount sufficient to positively influence the resuspension process. Typically, this amount is from 10 to 95% by weight of solids in a composition containing the latex, preferably 20 to 80%, and more preferably about 60% by weight. In devices in which only a limited volume of sample or other fluid will pass over the composition to be resuspended, the latex particles and the soluble polyhydroxy compound will be present in amounts that can be readily resuspended in the amount of liquid available. Typically, the resuspended latex particles will comprise no more than 10% of the total volume of particles and suspending fluid, preferably no more than 2%, and more preferably from 0.01 to 0.5%. While it is not necessary it is preferred that all of the polyhydroxy compound redissolve and all the latex resuspend. The quantity of additive should not be so high as to significantly reduce the assay response. In general, with assays requiring dilution, restrictions on the additive are minimal. Even when the additive changes the assay response, the assay medium can be adjusted to minimize any deleterious effect by varying the other components present in the system to obtain an optimal response.

The solubilizing sugar and/or sugar alcohol of the invention can be used with a variety of latex particles, especially those coated with antibody or antigen. The term latex generally refers to a suspension in water of particles of natural or synthetic rubber or plastic. Latex particles can be of any size but typically are from about 0.01 to about 2 microns in diameter. As far as the practice of the present invention is concerned, the chemical composition of the latex particle is relatively unimportant. There are numerous methods for attaching, either covalently or non-covalently, binding partners used in agglutination assays to the particle surface in order to render the particle satisfactory for use in an agglutination assay. As long as this attachment can take place, the chemical composition of the particle is usually irrelevant.

Many well known and commercially available latex particles are prepared by addition polymerization processes in aqueous media. Monomers utilized include acrolein, acrylate, methyl acrylate, methacrylate, methyl methacrylate, glycidyl methacrylate, styrene, vinyl toluene, t-butyl styrene, and copolymers containing mixtures of these monomers, the polymers and copolymers optionally containing crosslinking agents such as divinyl benzene and butadiene. Techniques for preparing such latex particles are well known. Likewise well known are surface modifications of latex particles, particularly those based on polystyrene and other aromatic molecules, in which amino, carboxyl, amide, hydrazine, and/or hydroxyl groups are introduced into the surface of the particle.

Numerous commercially available latex particles exist, including particles designed for use in peptide synthesis, chromatography, and diagnostic assays such as the agglutination assays previously described.

A number of U.S. Patents have issued which describe either particles that can be utilized in the present assay technique, binding partners that can be attached to the particles, and coupling methods for attaching the binding partners. These include U.S. Patent Nos. 4,064,080; 4,210,723; 4,264,766; 4,279,617; 3,857,931; 4,062,935; 4,138,213; 4,143,124; 4,162,895; 4,184,849; 4,307,190; 4,253,844; and 4,397,960. Some of these patents are listed elsewhere in this specification with reference to disclosures on specific topics.

The method of application of the latex composition to the solid surface on which it is dried and the method of drying the liquid-containing composition are not particularly limited. The liquid in which the latex is originally applied is typically aqueous, although water miscible solvents may be present. Other reagents, such as buffers, salts, proteins, detergents, bacteriostatic agents, and dyes, can and usually will be present in the application fluid. Vacuum drying, drying at ambient temperature in air or an inert gas (such as nitrogen or argon), drying at an elevated temperature (typically less than 500C) under air or an inert gas, drying under a stream of gas (air or inert gas), and various other techniques of drying are all suitable although certain techniques may be more suitable for particular manufacturing techniques. Any limitations that exist on the drying conditions will arise from the biological material that is present on the surface of the latex particle rather than from the suspending agents of the invention. For example, use of antibodies on the surface of the latex particles will require that temperatures be maintained in a range that will not irreversibly denature the proteinaceous antibodies (typically less than 700C, often less than 500C). The sugar and sugar alcohol molecules of the invention are typically stable to temperatures of at least 1000C. Generally it is desirable to complete the drying of protein in less than 30 minutes, preferably less than 15 minutes, more preferably less than 10 minutes.

The latex compositions of the invention can be used in any kind of agglutination assay. Agglutination assays can be used for the detection of any analyte which is a component of a multivalent binding pair or which competes with a component of a multivalent binding pair. By multivalent binding pair is meant two components which are capable of agglutination. In some cases, both binding partners will contain multiple binding sites. For example, the analyte can be a cell having multiple binding sites for an antibody and the binding partner can be the antibody (since antibodies have at least two binding sites for their antigen). This is the type of reaction that occurs in the classical case of agglutination of red blood cells. Bivalent antibodies cross-link type A or type B antigens present on the surface of red blood cells to cause agglutination of the cells.

However, it is not necessary that both members of the binding pair be multivalent. For example, a multivalent analyte (e.g., a polypeptide or carbohydrate containing repeating epitopes) can be reacted with a univalent antibody fragment or other binding protein provided that the univalent member is attached to the . particle.

Numerous multivalent binding pairs can be utilized in the practice of the present invention including but not limited to antibodies and antigens (which may themselves be antibodies or other binding components); hormones and receptors; enzymes and substrates; metabolites and carrier proteins; carbohydrates and lectins; complementary DNA, RNA, or DNA/RNA hybrid pairs; and cells (e.g., red blood cells, lymphocytes, and bacterial cells) and molecules that bind with cell membranes or components thereof, such as intercellular messengers and other molecules transportable across or capable of binding to the cell membrane. Because of the ease of forming antibodies, including monoclonal antibodies, capable of reacting with most antigens, antigen-antibody pairs are preferred multivalent binding pairs. Typically, the latex particles will have either the binding partner to the analyte or the analyte (or an analyte mimic) bound to its surface in order to carry out the agglutination assay.

Either component of a multivalent binding pair can be termed the analyte, with the remainder being the binding partner, depending on the analysis in question. For example, a diagnostic assay for infection of a human with a virus can be carried out by looking either for the viral antigens or for antibodies to the virus in the bloodstream of a patient. If the analyte is the viral particle itself, antibodies to the viral particle are bound to the solid latex particles of the invention. If the antibody is the analyte, either the (inactivated) viral particle, a part thereof containing the epitope that reacts with the antibody, or an artificially prepared epitope designed to imitate the binding site of the antibody can be the binding partner bound to the solid particle.

However, as indicated previously, the analyte does not have to be part of a multivalent binding pair. It can compete with a multivalent member if it is a member of a binding pair (not necessarily multivalent) and

4

an auxiliary agent which is multivalent is present. For example, an analyte can be a drug molecule, such as the theophylline. Antibodies capable of bonding theophylline can be present on the latex particles, and a multivalent reagent comprising theophylline analogs can be present in the system. Such a multivalent reagent could consist of a polymeric molecule having theophylline molecules attached at intervals along the polymer chain. If a sample not containing theophylline is added to a reagent consisting of the anti-theophylline-antibody-coated latex particles and the multivalent theophylline-containing polymer, agglutination of the latex particles will occur. However, if a sample containing theophylline is contacted with the reagent system, theophylline will react with the antibodies on the latex particles and inhibit reaction of the antibody with the multivalent analyte mimic. Decreasing amounts of agglutination will then correspond to increasing amounts of theophylline in the sample.

A number of techniques are available for attaching binding partners and other molecules (such as analyte mimics) to solid surfaces. These include physical adsorption onto the surface (which usually involves Van der Waal's interactions), ionic bonding between a charged surface and an opposite charge in the binding partner, and covalent bonds between the binding partner and the solid-particle surface. Such techniques are well known in the art and are described in K. E. Hechemy, "Latex Particle Assays in Laboratory Medicine" in Laboratory Management, June 1984, pp 27-40 and July 1984 pp 26-35; Martin, Biochem. (1981) 20:4229-4238; Merrifield, Science (1965) 150:1780; Lambris et al., J. Immunol. Methods. - (1983) 65:277-283; Limet, Op. Cit.; Stewart and Young, Solid Phase Peptide Synthesis, Pierce Chemical Company, Rockford, Illinois (1984); and Moussebois et al., U.S. Patent No. 4,397,960 Hager, U.S. Patent No. 3,857,931; Daniel, U.S. Patent No. 4,064,080; Dorman et al., U.S. Patent No. 4,210,723; Fischer, U.S. Patent 4,264,766; Masson et al., U.S. Patent No. 4,279,617; and Masson et al., U.S. Patent No. 4,253,844. Exemplary techniques include use of bifunctional bridging molecules which react at one end with a functional group present on the solid surface, such as a carboxylic acid group (which itself may be produced by oxidation of most organic surfaces), and react at the other end with a functional group present in the binding partner, which will typically be an amino acid or carbohydrate residue. Reactive amino acid side groups include carboxylate groups (e.g., aspartate), hydroxyl groups (e.g., tyrosine), sulfhydryl groups (e.g., cysteine), and amino groups (e.g., lysine). Reactive carbohydrate groups include aldehyde, hydroxyl, and carboxylic acid groups. Typical bridging groups include phenyl diisocyanates, bisdiazotized benzidine, diacid chlorides, dialdehydes, diamines, dihydroxyls, diacids, and molecules having any combination of two of these functional groups. Covalent coupling is preferred since small amounts of "free" binding partner which desorb from the particles can reduce assay sensitivity.

The following general procedure can be utilized in preparing latex particles for use in carrying out the present invention. Additional details are provided in the specific examples that follow. This general procedure is described in terms of utilizing antibody-coated latex particles in an assay for the antigen to which the antibody binds.

Commercial antisera or monoclonal antibodies can be utilized if desired. Antisera or monoclonal antibodies produced specifically for use in the practice of the present invention can also be utilized. Standard techniques are available for determining specificity of antibody in reference to agglutination assays; see National Committee for Clinical Laboratory Standards, Vol. III, No. 3 (1983) pp 414-425. Antisera are preferably fractionated by DEAE-Sephacyl chromatography utilizing the procedure recommended by the manufacturer (Pharmacia). The pervaporated product is dialyzed to a concentration of 10-20 mg/ml in phosphate-buffered saline. The suitability of a particular antiserum or batch of monoclonal antibodies can be verified utilizing a visible latex agglutination test. One such procedure is described in Kador et al. Am. J. Clin. Path. (1977) 68:284-289.; also see Hager, U.S. Patent No. 3,857,931.

When latex particles are utilized in agglutination assays, they are often "cleaned" prior to use since many latex particles as produced by their manufacturer contain sodium dodecylsulfate (SDS) on their surface. A suitable cleaning procedure utilizes AG 501-X8(d), manufactured by Bio-Rad Laboratories. A 10% w/v mixture of latex particles and water is mixed for two hours with AG 501-X8(d) which has been thoroughly washed with distilled water. Twice as much cleansing resin is utilized as latex. After incubation, the latex is filtered through glass wool to remove the resin and adjusted to approximately 4% solids with distilled water.

The process of coating the latex particles with antibody typically takes place in a buffer solution. A satisfactory buffer contains glycine, pH 8.4, 0.015 M (prepared using the minimum amount of sodium hydroxide) and merthiolate at a concentration of 0.2 mg/ml.

The following procedure is described for latex particles 0.30 micron in diameter. For particles with diameters other than 0.30 micron, the concentration of reagents is usually adjusted to reflect the total latex surface area that is present. For a constant weight of latex, the total surface area is inversely proportional to diameter so that a 0.60 micron latex utilizes half as much antibody or coating protein as 0.30 micron particles if both are at 2.5 mg/ml latex. It should also be remembered that antibody concentrations for optimal sensitivity will vary with different antibodies and with different batches of antisera. Other factors to keep in mind during the coating process include maintaining low salt levels (below 0.005 M) during the antibody binding in order to prevent clumping of latex. Flocculation resistance improves after a binding protein is coated on the latex particles.

Coating of latex particles with antibody can be carried out by mixing, in order, an aqueous suspension containing from 0.5 to 10%, preferably about 1% (w/v) of latex particles in water with buffer and an aqueous buffered solution of the antibody that is to be coated on the latex surface. Rapid mixing for a few seconds to a few minutes distributes the proteins, after which the latex particles are allowed to stand at room temperature for a few minutes. The coated particles are then mixed with an aqueous solution of a non-specific protein in order to coat latex surfaces to which antibody has not bound. One such typical procedure involves (after antibody has been attached) first coating with bovine caseins followed by coating with bovine serum albumin.

The agglutination assay in which the present method can be practiced is not limited; i.e., the solubilizers being used with any analyte (other than an assay for the solubilizer) in any sample which can be utilized in a binding assay. This includes but is not limited to physiological fluids, such as blood, blood hemolysate, serum, plasma, urine, feces, lymph fluid, spinal fluid, sputum, and the like. Fluids of non-biological origin can also be utilized as samples, such as waste treatment effluents, water samples, air samples, plant extracts, and the like. Numerous techniques have been developed for utilizing these and other sample types in diagnostic assays that employ binding reactions.

In some cases, an antigen extraction procedure is carried out prior to reaction of the reagent of the invention with the analyte. For example, strep A antigens are typically extracted into sodium nitrite / acetic acid / water prior to incubation reagent in a typical flocculation assay. Such extraction procedures are outside the scope of the present invention but may be utilized to improve sensitivity, to remove antigen from the sampling device (such as a throat swab), or for any other reason for which an extraction procedure is normally carried out.

As indicated previously, the method of the invention is particularly useful when carried out in small diagnostic devices made of hydrophobic plastic (or other hydrophobic materials) that contain internal chambers and capillary tracks of small volume in which the dried latex reagents are located. The device will typically be fabricated from materials with the appropriate physical properties, such as optical transmission, thermal conductivity, and mechanical properties, and which allow for uniform coating and stability of reagent, as well as medium compatibility, for example, blood compatibility. For this purpose, suitable plastics include those for high surface free energies and low water sorption, including PETG, polyester (Mylar$^R$), polycarbonate (Lexan$^R$), polyvinyl chloride, polystyrene, and SAN. A particularly preferred plastic is acrylonitrile-butadiene-styrene (ABS), particularly ABS supplied by Borg Warner under the tradename Cycolac. However, since these plastics are hydrophobic and exhibit poor reagent coating and poor blood flow, the plastics can be rendered more hydrophilic by treatment with argon plasma, using a plasma etcher or corona discharge (if desired). Suitable conditions are 10-25 watts at 13.56 MHz and one torr chamber pressure for 5-10 min. Alternatively, a protein, e.g., albumin coating, can be used in some instances by passing a solution through the device having from about 1-5% serum albumin, allowing the solution to stand for 30 min., wiping and drying. Other modifications may also find application. Plasma etching and corona discharge provide markedly superior flow control characteristics and reproducibility.

The sample will then be introduced into the device through an inlet port, which may introduce the sample into a chamber or a capillary. The sample will then transit the device passing through the capillary-(ies) or chamber(s), where the sample will encounter one or more reagents. In the present case, at least one of the reagents is the dried latex composition of the invention containing the polyhydroxy resuspending agent. By having orifices which connect the pathway to the atmosphere at one or more sites, one can terminate the flow up to that site, so that the medium may be incubated for various times or movement stopped subject to the initiating movement, for example, immediately prior to measurement. Mixing may be provided if desired in appropriate chambers, although mixing is not required to resuspend the latex particles, as previously mentioned. Valves may also be used to control fluid flow, if desired.

Additional information on the production of the analytical devices in which the present invention is preferably practiced can be found in the previously listed patents and patent applications.

Any liquid sample may be employed in these devices, and the liquid sample can be used to reconstitute the latex reagent. The device can be modified as necessary to provide a reasonable rate of flow due to the pumping of the capillary action, if capillary action is the sole motive force. It is to be understood that capillary action is the sole driving force in many (but not all) applications, relying on the surface action between the surface and the fluid. Where the sample is too viscous, it should be diluted to provide for a capillary pumping rate which allows for the desired manipulations, such as mixing, and for a reasonable flow time which will control the time period for the assay.

The sample may be derived from any source, such as a physiological fluid, e.g., blood, saliva, ocular lens fluid, cerebral spinal fluid, pus, sweat, exudate, urine, milk, or the like. The fluid may be subjected to prior treatment, such as preparing serum from blood, diluting pus, saliva, or the like; the methods of treatment may involve concentration, by filtration, distillation, dialysis, or the like; dilution, filtration, inactivation of natural components, concentration, chromatography, addition of reagents, chemical treatment, etc.

Besides physiological fluids, other liquid samples may be employed where the component(s) of interest may be either liquids or solids and the solid(s) dissolved in a liquid medium. Samples of interest include process streams, water, soil, plants or other vegetation, air, and the like.

Additionally or alternatively, a diluent that reconstitutes the latex reagent may be provided in liquid form in glass ampules. The liquid reagent could be released from the ampules by mechanical rupture. In such cases the diluent is not limited to being the sample but can be present in the form of a prepared reagent of any defined composition.

The analytes of interest are widely varied depending upon the purpose of the assay and the source. Analytes may include small organic molecules, such as drugs, hormones, steroids, neurotransmitters, growth factors, commercial chemicals, degradation products, drugs of abuse, metabolites, catabolites, etc. Large organic molecules may be determined, such as nucleic acids, proteins, polysaccharides, or the like. Aggregations of molecules may also be of interest, particularly naturally occurring aggregations such as viroids, viruses, cells, both prokaryotic and eukaryotic, including unicellular microorganisms, mammalian cells such as lymphocytes, epithelial cells, neoplastic cells, and the like.

In order to minimize the handling of reagents by the user of the device, all reagents may be supplied within the device, whereby mixing with the reagents occurs in the device. This includes not only the latex reagents that are the focus of the present invention but any other reagents as well that are present for other purposes in the same device. The reagents may be present either diffusively or non-diffusively bound to the surface of the device, that is, adhered, absorbed, adsorbed or covalently-linked, so that the reagent may become dissolved in the fluid or may remain fixed to the surface. In many instances it is essential that the reagent be present in a defined area or reaction chamber, which makes fabrication of an internal chamber followed by later addition of reagent virtually impossible.

The same or different reagent may be present in various locations throughout the device so that successive reactions can occur or a reagent continually supplied into the moving medium. Also, one could have a plurality of chambers and capillary channels. Frequently, the first unit will have a reactant. The chambers can be varied in size and purpose, providing the varying incubation times, varying reaction times, mixing of media from different capillaries, or the like. Any number of chambers may be employed, usually not exceeding six, more usually not exceeding about four, where the chambers may be in series or parallel. The size of the unit, either capillary or chamber, can be particularly important, where the reagent is fixed, so that the residence time in contact with the reagent will be affected by the area of the reagent contacted by the assay medium.

Although the present invention can be practiced in systems that allow for visual examination of the agglutination process, devices of the preferred type as described here readily allow use of instrumental techniques, and proper resuspension of the dried latex reagent is particularly important in such systems. Measurement of light, e.g., scatter, can be used to measure a change in the size population (agglutination). Turbidimetric movement of agglutination of small latex particles is preferred. Such measurements are well known. A laser is able to distinguish size of moving particles in a capillary track without a change in the flow rate. Small particles interrupt or otherwise interact with a laser beam with a high frequency and a low amplitude; large particles (agglutinated particles) have a lower frequency (fewer total particles) and a higher amplitude (each particle is larger). Thus the change in particle size distribution may be detected by integrated noise employing known circuitry. However, such an analysis would be severely disrupted if the latex reagent does not properly reconstitute upon the addition of diluent to the dry reagent.

The following examples are set forth for purposes of illustration only and are not to be considered limiting of the invention unless otherwise stated.

EXAMPLE 1

7

## Preparation of Theophylline IgG Conjugate

Theophylline-8-butyric acid lactam (Calbiochem) was conjugated to protein via a modification of the method described by Cook, C.E., et al., Res. Commun. Clin. Path. & Pharm. (1976) 13:497-505. Bovine IgG (1 mL) was dissolved in 100 mM sodium bicarbonate buffer, pH 8.5, at 30 mg/mL. With stirring and cooling in an ice bath, the lactam (0.5 mL x 0.7 mg/mL dissolved in dioxane) was added in one portion to the protein solution. The solution was incubated for four hours at 4° C and then dialyzed against four changes of 100 volumes of bicarbonate buffer to remove unconjugated reagent.

## EXAMPLE 2

## Cleaning of Small Latex Particles

To 10 mL x 10% w/v polystyrene latex (Seragen; 85 nm diameter) was added 4 g Biorad Ag 50W-X4 mixed bed resin. After 4h continuous mixing, the latex was recovered by filtration through glass wool.

## EXAMPLE 3

## Preparation of Antibody-Coated Small Latex Particles by Adsorption

Cleaned polystyrene latex (1% in 25 mM glycine, adjusted to pH 9.3 with NaOH, containing 0.01% sodium azide) was added with stirring to an equal volume of antibody solution (2 mg/mL) in 50 mM glycine (adjusted to pH 9.3 with NaOH) containing 6 mM NaCl. After stirring at room temperature for 15h, the antibody-coated latex was recovered and washed twice by centrifugation (60min. x 27,000g) into 10 mM sodium glycine, pH 9.3 containing 1% bovine serum albumin.

## EXAMPLE 4

## Preparation of Antibody-Coated Small Latex Particles by Covalent Attachment

To 19 mL of a 1.1% suspension of reactive latex (Duke Scientific, 101 nm diameter) was added 59 mL of 5 mM $K_2CO_3$, pH 9.0. A solution of antibody (9 mL x 3 mg/mL in 20 mM $K_2CO_3$, pH 9.0) was rapidly added to the latex suspension with stirring to give a ratio of 0.2 μg antibody/$cm^2$ latex surface area. Exactly one minute after antibody addition was complete, 13 mL x 400 mM glycine (adjusted to pH 9.3 with NaOH) was rapidly added with stirring. The resulting mixture was incubated at 37° for 24h. Antibody-coated latex was collected and washed by repeated centifugation in 50 mM glycine (pH 9.3). The same method was used to attach other proteins, including theophylline-labeled IgG, to small latex particles.

## EXAMPLE 5

## Preparation of Hepatitis-Virus-Core-Antigen-Coated Large Latex Particles

A glass vial was coated with 1% polyethylene glycol (M = 4,000) dissolved in glycine-buffered saline (0.1 M glycine (Na$^+$) containing 0.13 M NaCl, pH 8.4). After more than 5 minutes, the liquid was removed and 1 mM X 1% (w/v) 0.77-μ-diameter polystyrene latex beads (Seragen) added to the vial. Hepatitis B virus core antigen (Biogen, cloned material; 33 μL X 1.0 mg/mL) was added and mixed continuously for 1 hour at room temperature. The antigen-coated latex was recovered and washed by centrifugation and resuspension in a solution of bovine serum albumin (Sigma) in glycine-buffered saline. After mixing for 30 minutes at room temperature, the particles were again recovered and washed without incubation.

## EXAMPLE 6

### Assay of an Antibody-Coated Reagent Latex

An agglutinating polymer was made by covalent attachment of about nine molecules of a peptide antigen to polyaspartic acid (M = 15,000). A latex reagent was made by adsorption of a monoclonal antibody (which binds to the peptide antigen) to small latex particles (85 nm diameter) by adsorption. The assay of the agglutinating polymer was performed by mixing polymer and antibody-coated latex, then measuring the rate of increase of turbidity as follows: To 0.86 mL of 0.2 M glycine (Na$^+$) pH 9.0, containing 0.05% bovine serum albumin and 0.1% sodium azide, was added in rapid succession 0.45 mL of a suspension of antibody-coated latex (0.45% w/v) in the same solvent and 0.045 mL of agglutinator peptide (10 μg/mL) in 20 mM sodium phosphate, pH 6.0 containing 5 mM sodium azide and 0.1% bovine serum albumin. The mixture was aspirated into a temperature controlled flow cell of a spectrophotometer with a 1 cm optical path length and rapidly heated to 370C. The change of apparent absorbance at 540 nm was then recorded over 20s.

## EXAMPLE 7

### Drying of Latex Reagents onto Plastic Surfaces

A suspension of latex (0.09 mL x (typically) 0.45%) in 0.2 M glycine (Na$^+$) pH 9.0 containing a 0.05% bovine serum albumin and 0.1% sodium azide was applied to a piece of (1 x 0.5 cm) of acrylonitrile-butadiene-styrene plastic (cut from Biotrack PT cartridges). For most experiments, various additives were also present in the latex suspension (see Examples 9 and 10). Liquid was removed by exposure to desiccant under a variety of conditions (flowing air, flowing dry $N_2$, or vacuum). A dry film of reagent coated on the plastic resulted.

## EXAMPLE 8

### Measurement of the Recovery of Antibody-Latex Reagents After Drying on Plastic

Latex particles prepared as in Example 4 were dried as in Example 7. The assay method described in Example 6 was used for control (liquid reagent) assays, and was modified as follows for dried reagents: The plastic chip with dry latex was suspended in 0.95 mL of the glycine buffer of Example 6, then agglutinating peptide was added and the assay performed as described in Example 6.

## EXAMPLE 9

Effect of Sucrose on Recovery of Dry Antibody Latex

Measurements of the effect of sucrose on recovery of dry antibody latex were made according to Example 8 and are shown in Table 1.

This experiment demonstrates that the latex reagent remains attached to plastic after drying and rehydration unless (a) powerful ultrasonic vibration is used to dislodge the particles or (b) a compound of the invention is present in the latex formulation.

## EXAMPLE 10

### Comparison of Additives as Agents to Promote Re-suspension of Dry Antibody Latex

Measurements were carried out as in Example 8. Additives were added to the latex suspension at the indicated concentrations prior to drying. Recovery is given in Table 2 as a percentage of the activity of a non-dried (liquid) control. In general, sugar and sugar alcohol prevented loss of (recovered) activity caused by drying.

TABLE 1

| Recovery of Activity: Antibody-Latex | | | |
|---|---|---|---|
| Latex Suspending Medium Prior To Drying | Mixing Method[1] | Drying Method[2] | Activity Recovered Percent |
| Glycine Buffer[3] | V | Liquid | (100) |
| | V | Air | 0 |
| | A | Air | 93 |
| Glycine Buffer | V | Liquid | (100) |
| | B | Air | 87 |
| Glycine Buffer +5% Sucrose | V | Liquid | (100) |
| | V | Vac | 100 |
| | V | N$_2$ | 54 |
| | V | 370C | 83 |
| | V | Air | 67 |

1. Samples were either vortexed for 5s (V), sonicated in an ultra-sonic waterbath (A) for 5s, or placed directly on top of an ultrasonic horn (B) for 5-10s.

2. Drying method. Liquid: control samples run with antibody-latex in liquid form. Air: samples dried overnight in a desiccator at room temperature. Vac: samples dried by vacuum desiccation for 20min. N$_2$: samples dried y dry nitrogen flow at 370C: samples dried by desiccation at 370C.

3. Glycine buffer: 200 mM glycine (Na+), pH 9.0 containing 0.05% bovine serum albumin and 0.1% sodium azide.

10

TABLE 2

Comparison of Additives

| Additive | Class | Concentration* (%)] | Activity (%) Ref = Lig |
|---|---|---|---|
| Alanine | Amino Acid | 5 | 0 |
| Cysteine | Amino Acid | 5 | 0 |
| Glycine | Amino Acid | 5 | 0 |
| Gum Arabic | Polymer | 5 | 0 |
| Hydroxypropyl methyl celluose (High mw) | Polymer | 2.5 | 0 |
| Hydroxypropyl methyl celluose (Low mw) | Polymer | 2.5 | 0 |
| Polyacrylamide | Polymer | 5 | 0 |
| Polyvinyl alcohol | Polymer | 5 | 0 |
| Polyvinyl pyrrolidine | Polymer | 4 | 0 |
| Dextran Sulfate | Polymer | 4 | 1 |
| Polyethlene glycol | Polymer | 4 | 1 |
| Dextran 153K | Polymer | 5 | 23 |
| Sodium Chloride | Salt | 5 | 0 |
| Sodium Phosphate | Salt | 5 | 11 |
| D-turanose | Sugar | 5 | 58 |
| D-maltose | Sugar | 5 | 82 |
| Trehalose | Sugar | 5 | 101 |
| Sucrose | Sugar | 5 | 104 |
| Lactose | Sugar | 5 | 78 |
| D-fructose | Sugar | 5 | 52 |
| Mannose | Sugar | 5 | 54 |
| Fucose | Sugar | 5 | 62 |
| L-sorbose | Sugar | 5 | 65 |
| D-ribose | Sugar | 5 | 67 |
| 2-deoxy-D-glucose | Sugar | 5 | 73 |
| D-arabinose | Sugar | 5 | 75 |
| D-xylose | Sugar | 5 | 92 |
| Glucose | Sugar | 4 | 99 |
| Maltotriose | Sugar | 5 | 101 |

TABLE 2 CONTINUED

| Additive | Class | Concen-tration* (%) | Activity (%) Ref = Liq |
|---|---|---|---|
| 35D-Gluconic Acid-Na+ | Sugar Acid Salt | 5 | 41 |
| Mannitol | Sugar Alcohol | 4 | 0 |
| Sorbitol | Sugar Alcohol | 5 | 5 |
| Ribitol | Sugar Alcohol | 5 | 70 |
| D-xylitol | Sugar Alcohol | 5 | 92 |
| L-arabitol | Sugar Alcohol | 5 | 94 |
| L-xylitol | Sugar Alcohol | 5 | 94 |
| D-arabitol | Sugar Alcohol | 5 | 101 |
| D-glucosamine-HCl | Sugar Amine Salt | 5 | 25 |

*This is the concentration in the aqueous suspension of latex applied to the plastic. In the dry composition, the additive comprises most of the weight.

EXAMPLE 11

Stabilization of Antibody-Coated-Latex by Drying onto a Plastic Surface with Additive

The reagent of Example 3 or 4 at a concentration of 2% w/v with additives as indicated were either maintained as a liquid dispersion or dried (as 6 X 3 µL dots) onto plastic as in Example 7 with 5% sucrose added prior to drying. After storage, as indicated, activity (as in Example 8) and particle size measurements (Brookhaven Instruments BI 90 particle-size analyzer) were made. The results are shown in Table 3.

12

TABLE 3

| Stabilization of Antibody-Coated-Latex by Drying onto a Plastic Surface with Additive | | | | | | | | |
|---|---|---|---|---|---|---|---|---|
| | | | STORAGE CONDITIONS | | SIZE | | ACTIVITY | |
| REAGENT | FORM | ADDITIVE | Temp0C | Time(days) | nm | % of initial | mA/s | % of initial |
| 3 | Liquid | None | 4 | 0 | 87 | (100) | 28.1 | (100) |
| 3 | Dry | None | 4 | 0 | No measurement | | 0 | 0 |
| 3 | Dry | 5% Sucrose | 4 | 0 | 114 | (100) | 21.5 | (100) |
| 3 | Dry | 5% Sucrose | 4 | 119 | 116 | 102 | 22.7 | 106 |
| 3 | Dry | 5% Sucrose | 37 | 119 | 104 | 100 | 22.5 | 105 |
| 4 | Liquid | 5% Sucrose, 6% NaCl | 4 | 0 | 116 | (100) | 23.5 | (100) |
| 4 | Liquid | 5% Sucrose, 6% NaCl | 4 | 3 | * | * | 0.9 | 4 |
| 4 | Dry | 5% Sucrose, 6% NaCl | 23 | 3 | 154 | 133 | 20.1 | 85 |

* Particles were so large that valid size measurements were not possible

This experiment demonstrates that the latex reagent when dried onto plastic in the presence of sucrose remains essentially unaggregated and fully active during prolonged storage even at elevated temperature. Further, the drying prevents aggregation which can occur in liquid form over 3 days (Table 3; reagent 4).

Note that the drying caused a small increase of assay particles size and a small loss of activity; however, no further change occurred on storage. Since the change was stable to storage, the change did not affect ability to use the dried reagent in later assays.

## EXAMPLE 12

### Effect of Sucrose on the Recovery of Dried Latex-Linked Reagents from Plastic Surfaces

Latex-linked reagents were prepared according to Example 4 with either protein coatings as indicated or no protein coating ("glycine-latex"). Theophylline-labeled bovine IgG was made as in Example 1. Reagents were dried onto plastic with or without addition of sucrose (5%) in the suspension applied as in Example 7 (using 4 x 7 $\mu$L drops), under a stream of dry nitrogen at 370C. Recovery of the theophylline-labeled IgG-coated latex was estimated as follows: to 50 $\mu$L of a blood hemolysate containing a 1:30 dilution of whole blood in 5% lubrol (a detergent) containing 5 mM sodium azide and 6 mM potassium ferricyanide was added the dried latex coated on a piece of plastic. Buffer (0.9 mL X 0.2 M glycine (sodium), pH 9.0 containing 0.05% bovine serum albumin and 0.1% sodium azide) and 50 $\mu$L anti-theophylline (Instrumentation Labs. reagent for theophylline assay, used undiluted). After mixing, the agglutination rate of the resuspended latex was measured as the change in apparent absorbance (1 cm, 565 nm) at 370C over 20s. Results for theophylline are shown in Table 4 (Section 1).

Recovery of the other latex-linked reagents (in a useful, i.e., homogeneous, suspension) was estimated by measurement of the turbidity of the mixture made by resuspending dried latex (or the equivalent volume of the corresponding latex suspension) in a final volume of 1.0 mL of buffer. The results are shown in Table 4 (Section 2). The latex dried without sucrose was eluted from the plastic as macroscopic flakes which gave little or no turbidity.

TABLE 4

| Section 1 | | | | | |
|---|---|---|---|---|---|
| RECOVERY OF ACTIVITY OF THEOPHYLLINE-LABELED-BOVINE-IgG-COATED LATEX | | | | | |
| | Particle Size | | Sucrose | | % Activity Recovered | |
| | (nm) | | | | | |
| | 100 | | Yes | | 98 | |
| | 100 | | No | | 0 | |
| | 140 | | Yes | | 110 | |
| | 140 | | No | | 0 | |
| Section 2 | | | | | . |
| RECOVERY OF ACTIVITY OF VARIOUS LATEX REAGENTS | | | | | |
| PROTEIN | PARTICLE SIZE | OD565 | | | RECOVERY (%) | |
| | (nm) | WET | DRY +SUCROSE | DRY -SUCROSE | +SUCROSE | -SUCROSE |
| None (Glycine) | 100 | 0.262 | 0.445 | 0.038 | 170* | 15 |
| Bovine IgG | 140 | 0.841 | 0.819 | 0.026 | 97 | 3 |
| Chick IgG | 140 | 0.605 | 0.613 | 0.017 | 101 | 3 |
| Fetuin | 140 | 0.619 | 0.649 | 0.027 | 105 | 4 |
| Bovine Albumin | 140 | 0.601 | 0.651 | 0.016 | 108 | 3 |
| Hemoglobin | 140 | 0.609 | 0.688 | 0.019 | 113 | 3 |

*Recovery values in excess of 100% are due to partial agglutination of the latex. This is not a problem provided the recovery is reproducible, as was the case in these experiments.

EXAMPLE 13

Effect of Sucrose on Resuspension and Activity of Hepatitis B Virus Core Antigen Coated Latex Dried Onto Plastic

Antigen coated latex (prepared as in Example 5) (1 µL x 1.0% w/v suspended in 0·1M glycine pH 8·4 containing 0·1 6 M NaCl and 3 mM Sodium Azide either with or without 5% sucrose) was coated on the surface of piece of PETG plastic. After drying under a stream of dry nitrogen, the piece was incorporated into a cartridge by lamination of a track excised from double sided adhesive tape (4 mill thick) as described in USP 4,756,884. To test the activity of the dry reagent films, serum samples containing or not containing antibodies to hepatitis virus core antigen were applied to the sample application site and the reaction between the sample and the reagent film observed as the sample flowed over the film and the mixture was drawn down the part of the track distal from the sample application site. A positive reaction was recorded if the latex particles were agglutinated at the end of the track.

| Additive | Agglutination | |
|---|---|---|
| | + sample | - sample |
| None | + | + |
| 5% Sucrose | + | - |

These results show that the sucrose prevented non-specific agglutination of the latex particles caused by drying the latex reagent onto plastic.

All publications and patent applications mentioned in this specification are indicative of level of skill of those skilled in the art to which this invention pertains and are herein incorporated by reference to the same extent as if each individual patent application and publication had been individually indicated to be incorporated by reference.

Although the foregoing invention has been described in some detail by way of illustration and example for purposes of clarity of understanding, it will be obvious that certain changes and modifications may be practiced.

## Claims

1. A method for stabilizing latex particles for resuspension, which comprises:
applying an aqueous composition containing said particles and a water soluble, solid, polyhydroxy sugar or sugar alcohol to a surface; and
drying said composition onto said surface.

2. The method according to claim 1, wherein said sugar or sugar alcohol is a $C_4$-$C_7$ monomeric aldose or ketose; a dimeric or trimeric sugar consisting of $C_4$-$C_7$ monomeric aldoses, ketoses, or mixtures thereof; or a $C_4$-$C_7$ monomeric fully oxygenated sugar alcohol.

3. The method according to claim 1 or claim 2, wherein said sugar or sugar alcohol comprises 10 to 95 percent by weight of solids in said composition.

4. The method of claim 1, claim 2 or claim 3, wherein said latex particles have a carbohydrate-containing antigen bound to their surfaces.

5. The method of claim 1, claim 2 or claim 3, wherein said latex particles have a protein bound to their surfaces.

6. The method of claim 5, wherein said protein is an antibody or antibody fragment.

7. An analytical test device comprising a plastic, hydrophobic housing having an internal chamber in which a measurement of agglutination is made, characterized in that it comprises:
a dry latex reagent in an internal chamber of said device, said reagent comprising latex particles and a water soluble, solid, polyhydroxy sugar or sugar alcohol.

8. The test device according to claim 7, wherein said sugar or sugar alcohol is a $C_4$-$C_7$ monomeric aldose or ketose; a dimeric or trimeric sugar consisting of $C_4$-$C_7$ monomeric aldoses, ketoses, or mixtures thereof; or a $C_4$-$C_7$ monomeric sugar alcohol.

9. The test device according to claim 7 or claim 8, wherein said sugar or sugar alcohol is a $C_4$-$C_7$ monomeric aldose or ketose.

10. The test device according to claim 7 or claim 8, wherein said sugar or sugar alcohol is sucrose, trehalose, maltose, xylose, glucose, maltotriose, arabitol, or xylitol.

11. The test device according to any one of claims 7 to 10, wherein said housing comprises polystyrene, acrylamide-butadiene-styrene, a polyester, polycarbonate, or polyvinyl chloride.

12. The test device according to any one of claims 7 to 11, wherein said device is configured to provide diluent to a location of said reagent in a single pass of said diluent past said location, whereby said reagent is resuspended in said single pass of said diluent.